# EUROPEAN PATENT APPLICATION

(11) **EP 4 653 455 A1**
(43) Date of publication of application: **26.11.2025**
(21) Application number: 24744295.7
(22) Date of filing: 17.01.2024
(51) Int. Cl.: C07K 14/705, C07K 19/00, C12N 15/12, C12N 15/62, C12N 15/85, C12N 5/10, A61K 35/17, A61P 35/00

(54) **MUTATED PD1 ECTODOMAIN FRAGMENT AND CAR CONTAINING SAME, AND NK CELL**

(30) Priority: 17.01.2023 CN 202310081474
(71) Applicant: METTA THERAPEUTICS CO., LTD, Shanghai 200023 (CN); Center for Excellence in Molecular Cell Science, Chinese Academy of Sciences, Shanghai 200031 (CN)
(72) Inventor: LIU, Xiaolong, Shanghai 200031 (CN); LI, Xinyue, Shanghai 200031 (CN); LIU, Haifeng, Shanghai 200031 (CN)
(74) Representative: Nederlandsch Octrooibureau
(86) International application number: PCT/CN2024/072746
(87) International publication number: WO 2024/153120

(57) **Abstract**

The present invention relates to a mutated PD1 ectodomain fragment and a CAR containing same, and an NK cell. Specifically, the present invention provides a polypeptide, which is the mutated PD1 ectodomain fragment containing an amino acid sequence as shown in SEQ ID NO: 1 or consisting of the amino acid sequence as shown in SEQ ID NO: 1. The CAR of the present invention contains the polypeptide. The present invention further provides the NK cell expressing the CAR. The tumor cell killing capability of the NK cell that expresses the CAR containing the mutated PD1 ectodomain fragment in the present invention is significantly stronger than that of an NK cell that expresses a CAR containing a wild-type PD1 ectodomain fragment.

## Description

### TECHNICAL FIELD

The present invention relates to mutated PD1 ectodomain fragment and CAR containing same, and NK cell.

### BACKGROUND

In normal physiological conditions, activation of the PD-1 signal can inhibit excessive inflammatory responses and prevent the onset of autoimmune diseases. However, in the tumor environment, the PD-1/PD-L1 signal serves as a vital pathway for immune evasion by tumor cells. Highly expressed PD-L1 protein can be detected in the blood and tumor sites of patients with various tumors, including lung cancer, breast cancer, melanoma, head and neck cancer, and lymphoma. In tumor patients, PD-L1 interacts with the PD-1 receptor in immune cells such as T and NK. The PD-1 receptor mediates the brake signals of immune cells such as T and NK. The PD-1 receptor contains two inhibitory domains in the cell, namely ITIM (immunoreceptor tyrosine-based inhibitory motif) and ITSM (immunoreceptor tyrosine-based switch motif), thereby inhibiting TCR signals/NK activation signals through phosphorylation of SHP protein. There are currently a variety of antibody therapies against PD-1/PD-L1, which have certain effects in tumors such as lymphoma and melanoma, but there are still drawbacks. On the one hand, antibody therapy can only block the interaction between immune cells and tumor cells in the tumor through antibody blocking, and cannot revers the immune cells that have already interacted with tumor cells thus still has an immunosuppressive effect. On the other hand, the Fc region of the antibody will largely induce effects like ADCC and ADCP with immune cells in patients, resulting in immune system disorders.

NK-CAR (Chimeric Antigen Receptor Natural Killer) cell technology is a novel cellular therapy, which is to reinfuse modified NK cells into the human body, directly kill tumors, activate the autoimmune system, and overcome the cytotoxicity, drug resistance, and recurrence defects of traditional treatment methods, and is considered to be one of the most effective treatment methods for malignant tumors. At present, NK-CAR technology has achieved remarkable results in hematological malignancies such as acute and chronic lymphocytic leukemia and lymphoma (such as B-cell lymphoma and acute and chronic B-lymphocytic leukemia).

NK-CAR cells have strong killing ability, but in the tumor microenvironment, PDL1 and others in the tumor microenvironment can inhibit the activity of killer NK cells through the receptor PD1 of immune cells, so that tumor cells can escape immune surveillance. Therefore, it is hoped to utilize the negative feedback signals from tumor cells (i.e., the PD-L1-PD-1 interaction) to activate immune cells, thereby reducing the inhibitory effect of tumor cells on immune cells while activating NK cells, which in turn enhances the killing effect of NK-CAR cells against tumor cells.

### SUMMARY

The present invention provides a polypeptide, comprising the amino acid sequence as shown by SEQ ID NO: 1, or consisting of the amino acid sequence as shown by SEQ ID NO: 1.

The present invention further provides a chimeric antigen receptor, and from the N-terminus to the C-terminus, the chimeric antigen receptor comprises sequentially linked: an optional signal peptide, the polypeptide described herein, a hinge region, a transmembrane domain, an intracellular costimulatory signaling domain, and an intracellular signaling domain.

In one or more embodiments, the signal peptide is selected from the group consisting of: a CD8 signal peptide, a CD28 signal peptide, or a CD4 signal peptide; preferably, the signal peptide is a CD8 signal peptide; preferably, the amino acid sequence of the CD8 signal peptide is shown by amino acid residues 1-20 of SEQ ID NO: 3.

In one or more embodiments, the hinge region is selected from the group consisting of: a CD8α hinge region, an IgD hinge region, an IgG1 Fc CH2CH3 hinge region, or an IgG4 Fc CH2CH3 hinge region; preferably, the hinge region is a CD8α hinge region; preferably, the amino acid sequence of the CD8α hinge region is shown by amino acid residues 171-225 of SEQ ID NO: 3.

In one or more embodiments, the transmembrane domain is selected from the group consisting of: a CD28 transmembrane domain, a CD8 transmembrane domain, a CD3ζ transmembrane domain, a CD134 transmembrane domain, a CD137 transmembrane domain, an ICOS transmembrane domain, or a DAP10 transmembrane domain; preferably, the transmembrane domain is a CD8 transmembrane domain; preferably, the amino acid sequence of the CD8 transmembrane domain is shown by amino acid residues 226-246 of SEQ ID NO: 3.

In one or more embodiments, the intracellular costimulatory signaling domain is an intracellular domain of a costimulatory signaling molecule, preferably selected from the group consisting of: the intracellular domains of CD28, CD134/OX40, CD137/4-1BB, lymphocyte-specific protein tyrosine kinase, inducible T-cell costimulator, or DNAX-activating protein 10; preferably, the intracellular costimulatory signaling domain is the intracellular domain of 4-1BB; preferably, the amino acid sequence of the intracellular domain of 4-1BB is shown by amino acid residues 247-288 of SEQ ID NO: 3.

In one or more embodiments, the intracellular signaling domain is a CD3ζ intracellular signaling domain or an FcεRIγ intracellular signaling domain; preferably, the intracellular signaling domain is a CD3ζ intracellular signaling domain; preferably, the amino acid sequence of the CD3ζ intracellular signaling domain is shown by amino acid residues 289-400 of SEQ ID NO: 3.

In one or more embodiments, the CAR comprises sequentially linked, from the N-terminus to the C-terminus, a CD8 signal peptide, the polypeptide of claim 1, a CD8α hinge region, a CD8 transmembrane domain, a 4-1BB intracellular domain, and a tyrosine activation motif of CD3ζ.

In one or more embodiments, the amino acid sequence of the chimeric antigen receptor is the amino acid residues 1-20 of SEQ ID NO:3, SEQ ID NO:1 and the amino acid residues 171-400 of SEQ ID NO:3, sequentially linked from the N-terminus to the C-terminus; or the amino acid sequence of the chimeric antigen receptor shown by SEQ ID NO:3.

The present invention further provides a nucleic acid molecule, encoding the polypeptide chimeric antigen receptor described herein; preferably, the nucleic acid molecule is a DNA molecule or an RNA molecule.

In one or more embodiments, the nucleic acid molecule is a coding sequence of the polypeptide of claim 1, and its nucleotide sequence is shown by nucleotides 70-510 of SEQ ID NO: 2.

In one or more embodiments, the nucleic acid molecule sequentially comprises, from the 5' terminus to the 3' terminus: the nucleotide sequence shown by nucleotides 1-60 of SEQ ID NO: 2 and the nucleotide sequence shown by nucleotides 70-1200 of SEQ ID NO: 2, or its nucleotide sequence is shown by SEQ ID NO: 2.

In one or more embodiments, the nucleotide sequence of the nucleic acid molecule is as shown by SEQ ID NO: 2.

The present invention further provides a nucleic acid construct, comprising the nucleic acid molecule described herein.

In one or more embodiments, the nucleic acid construct is an expression cassette that, in addition to the nucleic acid molecule, further comprise a regulatory sequence.

In one or more embodiments, the nucleic acid construct is a vector, comprising an expression vector and an integrated vector for integrating the nucleic acid molecule into the genome of the host cell; preferably, the vector is a transposon vector, and more preferably, the transposon vector is a eukaryotic expression vector containing transposable elements selected from the group consisting of: piggybac, sleeping beauty, frog prince, Tn5, or Ty.

The present invention further provides a host cell, comprising the nucleic acid construct described herein and/or expressing the polypeptide or CAR described herein; preferably, the host cell is an NK cell.

The present invention further provides a composition or kit, comprising the vector described herein and optionally a reagent for transfection.

In one or more embodiments, the kit comprises the composition.

The present invention further provides a pharmaceutical composition, comprising the NK cells described herein and a pharmaceutically acceptable carrier or excipient.

The present invention further provides use of the polypeptide, chimeric antigen receptor, nucleic acid molecule, nucleic acid construct, host cell, or pharmaceutical composition described herein in the manufacture of a medicament for treating or preventing cancers mediated by PD-1 or PD-L1; preferably, the cancers are selected from the group consisting of: gastric cancer, lung cancer (such as non-small cell lung cancer), liver cancer, intrahepatic cholangiocarcinoma, colon cancer, pancreatic cancer, ovarian cancer, breast cancer, cervical cancer, head and neck squamous cell carcinoma, nasopharyngeal carcinoma, esophageal cancer, bladder cancer, renal cell carcinoma, skin cancer, and oral squamous cell carcinoma.

### Description of drawings

Figure 1: Sequence of NK-PD1M-CAR targeting PD-L1.
Figure 2: Expression of CD56 and CAR in NK-PD1M-CAR. In the figure, the control ("control") refers to NK cells from the same batch without transfection of the coding gene for chimeric antigen receptor PD1M-CAR.
Figure 3: *In vitro* killing result of NK cells against the PD-L1-overexpressing K562 tumor cell line.
Figure 4: Killing result of NK+HAC and NK-PD1M-CAR against A549 cells. Based on the rightmost curves in the figure, from top to bottom, the groups correspond to: A549; A549+NK+HAC, E/T=0.625; A549+NK, E/T=1.25; A549+NK+HAC, E/T=1.25; A549+NK-PD1M-CAR, E/T=0.625; A549+NK-PD1M-CAR, E/T=1.25.
Figure 5: Killing result of NK-PD1WT-CAR and NK-PD1M-CAR against A549 cells. Based on the rightmost curves in the figure, from top to bottom, the groups correspond to: A549; A549+NK-GFP+HAC, E/T=0.625; A549+NK-PD1WT-CAR, E/T=0.625; A549+NK-PD1M-CAR, E/T=0.625; A549+NK-GFP+HAC, E/T=1.25; A549+NK-PD1WT-CAR, E/T=1.25; A549+NK-PD1M-CAR, E/T=1.25.
Figure 6: Killing result of NK-PD1WT-CAR and NK-PD1M-CAR against H1299 cells. Based on the rightmost curves in the figure, from top to bottom, the groups correspond to: H1299; H1299+NK-GFP+HAC; H1299+NK-PD1WT-CAR; H1299+NK-PD1M-CAR.
Figure 7: Killing result of NK-PD1WT-CAR and NK-PD1M-CAR against HCC827 cells. Based on the rightmost curves in the figure, from top to bottom, the groups correspond to: HCC827; HCC827+NK-GFP+HAC; HCC827+NK-PD1WT-CAR; HCC827+NK-PD1M-CAR.
Figure 8: Killing effect of NK-PD1M-CAR targeting HCC827 cells.
Figure 9: PD-L1 expression in several cell lines.
Figure 10: Expression of CD56 and CAR in NK-PD1WT-CAR. In the figure, the control ("control") refers to NK cells from the same batch without transfection of the coding gene for chimeric antigen receptor PD1WT-CAR.
Figure 11: Schematic diagram of the mechanism of CAR-NK cells *in vivo.*
Figure 12: *in vivo* imaging of mice.
Figure 13: Statistical analysis of *in vivo* imaging.
Figure 14: Tumor growth in mice on day 21 after reinfusion of different NK cells.
Figure 15: Survival curve of mice.

### DETAILED DESCRIPTION

It is to be understood that within the scope of the present invention, the various technical features of the present invention described above and the various technical features specifically described below (such as in the examples) can be combined with each other, thereby forming a preferred technical solution.

In the present invention, the term "expression cassette" refers to the complete elements required for the expression of a gene, including a promoter, a gene coding sequence, and a polyA tailing signal sequence.

The term "coding sequence" refers to the portion of a nucleic acid sequence that directly determines the amino acid sequence of its protein product (e.g., CAR). The boundaries of a coding sequence are typically determined by the ribosome binding site (for prokaryotic cells) immediately upstream of the open reading frame (ORF) at the 5' terminus of the mRNA and the transcription termination sequence immediately downstream of the ORF at the 3' terminus of the mRNA. Coding sequences may include, but are not limited to, DNA, cDNA, and recombinant nucleic acid sequences.

The term "costimulatory molecule" refers to molecules expressed on the surface of antigen-presenting cells that can bind to costimulatory molecule receptors on Th cells and generate costimulatory signals. The proliferation of lymphocytes requires not only the binding of antigens but also the reception of signals from costimulatory molecules. Costimulatory signals are primarily transmitted to T cells through the binding of costimulatory molecules CD80 and CD86 (expressed on the surface of antigen-presenting cells) to the CD28 molecule on the surface of T cells. B cells can receive costimulatory signals through general pathogen components (e.g., LPS), through complement components, or through CD40L on the surface of activated antigen-specific Th cells.

The term "linker" or "hinge" refers to a polypeptide fragment that link different proteins or polypeptides, with the aim of enabling the linked proteins or polypeptides to maintain their respective spatial conformations, thereby preserving the function or activity of the proteins or polypeptides. Exemplary linkers include those containing G and/or S, as well as, for example, the Furin 2A peptide.

The term "pharmaceutically acceptable excipient" refers to carriers and/or excipients that are pharmacologically and/or physiologically compatible with the subject and the active ingredient, which are well known in the art (e.g., Remington's Pharmaceutical Sciences, edited by Gennaro AR, 19th ed., Pennsylvania: Mack Publishing Company, 1995), and include but are not limited to: pH adjusters, surfactants, adjuvants, and ionic strength enhancers. For example, pH adjusters include but are not limited to phosphate buffers; surfactants include but are not limited to cationic, anionic, or nonionic surfactants (e.g., Tween-80); and ionic strength enhancers include but are not limited to sodium chloride.

The term "effective amount" refers to a dose that can achieve the treatment, prevention, alleviation, and/or relief of the disease or condition described in the present invention in a subject.

The term "disease and/or condition" refers to a physical condition of the subject, which is associated with the disease and/or condition described in the present invention.

The term "subject" or "patient" may refer to a patient or other animal, particularly a mammal (e.g., human, dog, monkey, cow, horse, etc.) that receives the pharmaceutical composition of the present invention to treat, prevent, alleviate, and/or ameliorate the disease or condition described in the present invention.

The term "chimeric antigen receptor" (CAR) refers to an artificially engineered receptor, which can anchor specific molecules that recognize antigens on the surface of tumor cells (e.g., antibodies) on immune cells (e.g., T cells), enabling immune cells to recognize tumor antigens or viral antigens and kill tumor cells or virus-infected cells. A CAR typically comprises, in sequence, an optional signal peptide, a polypeptide that binds to tumor cell membrane antigens, a hinge region, a transmembrane domain, and an intracellular signaling domain.

The present invention constructs a CAR using a mutated extracellular domain of PD1 and expresses this CAR in NK cells. The inventors have found that NK cells expressing the CAR containing the mutated PD1 extracellular domain exhibit significantly enhanced tumor cell killing ability compared to those expressing the CAR containing the wild-type PD1 extracellular domain, thereby completing the present invention.

Therefore, the first aspect of the present invention provides a mutated polypeptide, which is a mutant of the extracellular domain of PD1. In some embodiments, the polypeptide comprises the amino acid sequence shown by SEQ ID NO: 1, or consists of the amino acid sequence shown by SEQ ID NO: 1. The polypeptides of the present invention further comprise mutants with 1 to 8, preferably 1 to 5, more preferably 1 to 3 amino acid mutations (including insertions, deletions, and/or substitutions) compared to SEQ ID NO: 1. The mutations are preferably conservative substitutions well-known in the art, for example, replacing one or more (e.g., 1 to 8, 1 to 5, or 1 to 3) residues in SEQ ID NO: 1 with amino acid residues having the same or similar properties (e.g., classified by side chain groups or by chemical structure). It should be understood that the mutant of SEQ ID NO: 1 maintains biological activity of SEQ ID NO: 1, particularly the biological activity exhibited by SEQ ID NO: 1 herein.

The second aspect of the present invention provides a chimeric antigen receptor (CAR) comprising, a signal peptide, a mutant of the PD1 extracellular domain described herein, a hinge region, a transmembrane domain, an intracellular co-stimulatory signal domain and an intracellular signal domain linked in sequence from the N-terminus to the C-terminus.

A signal peptide is a short peptide chain (5-30 amino acids in length) that guides newly synthesized proteins to the secretory pathway. It often refers to the amino acid sequence at the N-terminus (sometimes not necessarily at the N-terminus) of a newly synthesized polypeptide chain that is used to guide the transmembrane transfer (localization) of proteins. It is responsible for directing proteins to subcellular organelles with different membrane structures within the cell. In some embodiments, the signal peptide herein may be selected from the group consisting of: CD8 signal peptide, CD28 signal peptide, or CD4 signal peptide. Preferably, the signal peptide is a CD8 signal peptide. Preferably, the amino acid sequence of the CD8 signal peptide is shown by amino acid residues 1-20 of SEQ ID NO: 3.

The hinge region refers to the region between the CH1 and CH2 domains of the immunoglobulin heavy chain. This region is rich in proline, does not form an α-helix, and is prone to stretching and a certain degree of twisting. In some embodiments, the hinge region used herein may be selected from the group consisting of: CD8α hinge region, IgD hinge region, IgG1 Fc CH2CH3 hinge region, and IgG4 Fc CH2CH3 hinge region. Preferably, the hinge region is a CD8α hinge region or an IgG4 Fc CH2CH3 hinge region. More preferably, the amino acid sequence of the CD8α hinge region is shown by amino acid residues 171-225 of SEQ ID NO: 3.

In some embodiments, the transmembrane domain herein may be one of the transmembrane domains of CD28, CD8, CD3ζ, CD134, CD137, ICOS, and DAP10. Preferably, the CD8 transmembrane domain is used herein; more preferably, the amino acid sequence of the CD8 transmembrane domain is shown by amino acid residues 226-246 of SEQ ID NO: 3.

In some embodiments, the intracellular costimulatory signaling domain used herein includes the intracellular domains of costimulatory signaling molecules, including those of CD28, CD134/OX40, CD137/4-1BB, lymphocyte-specific protein tyrosine kinase, inducible T-cell costimulator (ICOS), and DNAX activation protein 10. Preferably, the intracellular costimulatory signaling domain is the intracellular domain of 4-1BB. More preferably, the amino acid sequence of the intracellular domain of 4-1BB is as shown by amino acid residues 247-288 of SEQ ID NO: 3.

In some embodiments, the intracellular signaling domain used herein is the intracellular signaling domain of CD3ζ or FcεRIγ. Preferably, the CD3ζ intracellular signaling domain is used herein; more preferably, the amino acid sequence of the CD3ζ intracellular signaling domain is shown by amino acid residues 289-400 of SEQ ID NO: 3.

In some embodiments, the chimeric antigen receptor described herein comprises, in sequence from the N-terminus to the C-terminus, a CD8 signal peptide, the mutant of the extracellular domain of PD1 described herein, a CD8α hinge region, a CD8 transmembrane domain, the intracellular domain of 4-1 BB, and the tyrosine activation motif of CD3ζ.

The various components forming the chimeric antigen receptor herein, such as the signal peptide, the mutant of the extracellular domain of PD1, the hinge region, the transmembrane domain, the intracellular costimulatory signaling domain, and the intracellular signaling domain, may be directly linked to each other or linked via a linker sequence. The linker sequence may be a linker sequence well-known in the art for use with antibodies, such as a linker sequence containing G and S. The length of the linker may be 3 to 25 amino acid residues, such as 3 to 15, 5 to 15, or 10 to 20 amino acid residues. In certain embodiments, the linker sequence is a polyglycine linker sequence. The number of glycine residues in the linker sequence is not particularly limited, but is typically 2 to 20, such as 2 to 15, 2 to 10, or 2 to 8. In addition to glycine and serine, the linker may further contain other known amino acid residues, such as alanine (A), leucine (L), threonine (T), glutamic acid (E), phenylalanine (F), arginine (R), glutamine (Q), and the like.

It should be understood that in the process of genetic cloning, it is often necessary to design appropriate restriction enzyme sites (e.g., amino acid residues 21-23 of SEQ ID NO: 3), which inevitably introduces one or more irrelevant residues at the terminus of the expressed amino acid sequence. However, this does not affect the activity of the target sequence. Additionally, to construct fusion proteins, promote the expression of recombinant proteins, obtain recombinant proteins that are automatically secreted outside the host cell, or facilitate the purification of recombinant proteins, it is often necessary to add certain amino acids to the N-terminus, C-terminus, or other suitable regions in the recombinant protein. Therefore, the amino terminus or carboxy terminus of the CAR herein, or between its various elements, may further contain one or more polypeptide fragments serving as protein tags. Any suitable tag can be used herein. For example, the tags may include FLAG, HA, HA1, c-Myc, Poly-His, Poly-Arg, Strep-Tagll, AU1, EE, T7, 4A6, ε, B, gE, and Ty1. These tags can be used for protein purification.

In some embodiments, the amino acid sequence of the chimeric antigen receptor described herein consists of amino acid residues 1-20 of SEQ ID NO: 3, SEQ ID NO: 1, and amino acid residues 171-400 of SEQ ID NO: 3, linked in sequence from the N-terminus to the C-terminus. In some embodiments, the amino acid sequence of the chimeric antigen receptor described herein is as shown by SEQ ID NO: 3.

The present invention further provides a nucleic acid molecule, which encodes the mutated polypeptide or chimeric antigen receptor described in any embodiment herein. The nucleic acid molecule can be a DNA molecule or an RNA molecule. In some embodiments, the nucleic acid molecule is an mRNA molecule. DNA can be single-stranded or double-stranded.

The nucleic acid molecules described herein can generally be obtained by PCR amplification. Specifically, primers can be designed based on the nucleotide sequences disclosed herein, and the relevant sequences can be amplified using a commercially available cDNA library or a cDNA library prepared by routine methods known to those skilled in the art as a template. When the sequence is long, PCR amplification is often required to be performed twice or more times, and then the amplified fragments from each step are spliced together in the correct sequence.

In some embodiments, the nucleic acid molecule is the coding sequence of the polypeptide described herein; preferably, its nucleotide sequence is shown by nucleotides 70-510 of SEQ ID NO: 2.

In some embodiments, the nucleic acid molecule comprises, in sequence from the 5' terminus to the 3' terminus, the nucleotide sequence shown by positions 1-60 of SEQ ID NO: 2 and the nucleotide sequence shown by positions 70-1200 of SEQ ID NO: 2. In some embodiments, the nucleotide sequence of the nucleic acid molecule is shown by SEQ ID NO: 2.

In some embodiments, the present invention further provides a nucleic acid construct, comprising the nucleic acid molecule described in any embodiment herein. In some embodiments, the nucleic acid construct is an expression cassette, in addition to the nucleic acid molecule, further comprising a regulatory sequence.

The regulatory sequence may be a suitable promoter sequence. The promoter sequence is typically operably linked to the coding sequence of the protein to be expressed. The promoter may be any nucleotide sequence that exhibits transcriptional activity in the selected host cell, including mutated, truncated, and hybrid promoters, and may be obtained from genes encoding extracellular or intracellular polypeptides that are either homologous or heterologous to the host cell.

The regulatory sequence may also be a suitable transcription terminator sequence, which is a sequence recognized by the host cell to terminate transcription. The terminator sequence is operably linked to the 3' terminus of the nucleotide sequence encoding the polypeptide. Any terminator that is functional in the selected host cell can be used herein.

In certain embodiments, the nucleic acid construct is a vector. Specifically, the coding sequence of the CAR herein can be cloned into many types of vectors, including but not limited to plasmids, phagemids, phage derivatives, animal viruses, and cosmids. The vector can be an expression vector or an integrating vector used to integrate the nucleic acid molecule described herein into a host cell. The expression vector can be provided to cells in the form of a viral vector. Viruses that can be used as vectors include, but are not limited to, retroviruses, adenoviruses, adenoviruses, herpesviruses, and lentiviruses.

Typically, a suitable vector contains an origin of replication functional in at least one organism, a promoter sequence, convenient restriction enzyme sites, and one or more selectable markers. For example, in certain embodiments, the present invention uses a retroviral vector, which contains an origin of replication, 3' LTR, 5' LTR, the coding sequence of the CAR described herein, and an optional selectable marker.

Suitable promoters include, but are not limited to, the immediate early cytomegalovirus (CMV) promoter sequence. This promoter sequence is a strong constitutive promoter sequence capable of driving high-level expression of any polynucleotide sequence operably linked thereto. Another example of a suitable promoter is the elongation factor-1α (EF-1α). However, other constitutive promoter sequences may also be used, including but not limited to the simian virus 40 (SV40) early promoter, the mouse mammary tumor virus (MMTV) promoter, the human immunodeficiency virus (HIV) long terminal repeat (LTR) promoter, the MoMuLV promoter, the avian leukemia virus promoter, the EB virus immediate early promoter, the Rous sarcoma virus promoter, and human gene promoters such as, but not limited to, the actin promoter, myosin promoter, heme promoter, and creatine kinase promoter. Additionally, inducible promoters may also be considered. The inducible promoter provides a molecular switch that can turn on the expression of a polynucleotide sequence operably linked to the inducible promoter when expression is desired and turn it off when expression is not desired. Examples of inducible promoters include, but are not limited to, the metallothionein promoter, glucocorticoid promoter, progesterone promoter, and tetracycline promoter.

Optional markers include either or both of marker genes and reporter genes to facilitate the identification and selection of expressing cells from a population of cells infected with the viral vector. Useful selectable marker genes include, for example, antibiotic resistance genes such as neo. Optional reporter genes may include genes encoding luciferase, β-galactosidase, chloramphenicol acetyltransferase, secreted alkaline phosphatase, or green fluorescent protein.

In certain embodiments, the coding sequence of the chimeric antigen receptor (CAR) described herein is cloned into a vector (also known as an integrating vector) for integrating a nucleic acid sequence of interest into the genome of a host cell, particularly a transposon vector. Suitable transposon vectors are eukaryotic expression vectors containing transposable elements selected from the group consisting of: piggybac, sleeping beauty, Frog Prince, Tn5, or Ty. Such transposon vectors contain the 5' inverted terminal repeat (5' LTR) and the 3' inverted terminal repeat (3' LTR) of the corresponding transposon. The transposase can be derived from the piggyBac, Sleeping Beauty, Frog Prince, Tn5, or Ty transposon system transposase. When a transposase from a different transposon system is used, the sequences of the 5' LTR and 3' LTR in the vector are correspondingly changed to sequences compatible with that transposon system, which can be readily determined by those skilled in the art. Between the 5' LTR and 3' LTR is the expression cassette of the CAR of the present invention, including the corresponding promoter sequence, the coding sequence of the CAR, and a polyA tailing signal sequence.

The present invention further provides a host cell, which contains the nucleic acid construct described herein and/or expresses the CAR described herein. In some embodiments, the host cell is a NK cell.

Techniques well known in the art can be used to transfect the nucleic acid construct of the present invention into host cells. Transfection methods are routine in the art, including but not limited to: viral transduction, microinjection, particle bombardment, gene gun transformation, and electroporation, etc. In certain embodiments, electroporation is used to transfect the vector into the cells of interest.

The present invention further provides a composition, which contains a vector comprising the chimeric antigen receptor expression cassette described herein. The composition further contains suitable reagents, including but not limited to transfection reagents.

The present invention further provides a kit, which contains a vector comprising the chimeric antigen receptor expression cassette described herein, or contains the composition described herein. The kit may further be equipped with reagents or instruments for transfecting the vector into cells.

The present invention further provides a pharmaceutical composition, which contains the NK cells described herein. The pharmaceutical composition may contain suitable pharmaceutically acceptable carriers or excipients. The pharmaceutical composition contains a therapeutically or prophylactically effective amount of NK cells. The therapeutically or prophylactically effective amount of NK cells can be determined according to factors such as the patient's condition.

The present invention further provides the use of the mutant of the PD1 extracellular domain, CAR, its coding sequence or complementary sequence, nucleic acid construct, and host cell described herein in the preparation of a medicament for treating or preventing cancer. The present invention further provides the mutant of the PD1 extracellular domain, CAR, its coding sequence or complementary sequence, nucleic acid construct, and host cell described herein for use in treating or preventing cancer. The present invention further provides a method for treating or preventing cancer, which comprises administering to a subject in need thereof a therapeutically or prophylactically effective amount of the NK cells or pharmaceutical composition thereof of the present invention. The cancer described herein is preferably a PD1- or PDL1-mediated cancer, i.e., a cancer that can be treated or prevented by blocking the signaling pathway between PD1 and PDL1, or a disease or condition caused by or characterized by PD-L1 expression, including T cell dysfunctional diseases such as cancer and inflammatory diseases. In some embodiments, the cancers of the present invention include, but are not limited to, gastric cancer, lung cancer (e.g., non-small cell lung cancer), liver cancer, intrahepatic cholangiocarcinoma, colon cancer, pancreatic cancer, ovarian cancer, breast cancer, cervical cancer, head and neck squamous cell carcinoma, nasopharyngeal cancer, esophageal cancer, bladder cancer, renal cell carcinoma, skin cancer, and oral squamous cell carcinoma.

The present invention will be further illustrated with reference to specific examples. It should be understood that these examples are only used to illustrate the present invention and not to limit the scope thereof. For the experimental methods in the following examples where specific conditions are not specified, they are usually carried out under conventional conditions or according to the conditions recommended by the manufacturer. Unless otherwise specified, percentages and parts are calculated by weight.

### Example 1: Preparation of UCB-NK

### (1) Isolation of CBMC

One unit of umbilical cord blood was diluted with three volumes of PBS containing 2% EDTA. For every 35 mL of diluted cord blood, 10 mL of Ficoll separation medium was carefully added vertically from the bottom using a pipette. The mixture was centrifuged at 400g for 35 minutes with an acceleration rate of 1 and a deceleration rate of 0. After centrifugation, the cells separated into layers, and the intermediate lymphocyte layer was collected. The collected cells were washed with PBS to obtain CBMC.

### (2) Purification of NK cells by depleting CD3+ cells

The CBMC obtained above were resuspended in PBS containing 1% EDTA to prepare a cell suspension. According to the kit instructions, depletion antibodies and magnetic beads were sequentially added, followed by a 5-minute incubation. The incubated cells were then subjected to magnetic separation using a magnet. The cells were washed with PBS. After the immunomagnetic beads were removed, CD3-negative lymphocytes were obtained.

### (3) Purification and maturation of NK Cells

The CD3-negative lymphocytes obtained by immunomagnetic separation were cultured. On day 5 of culture, cell counting and medium replacement were performed by centrifugation. The cell concentration was adjusted to 1×10⁶ cells/mL, and the cells were seeded for further culture. On day 7, cell condition was observed. If the cell density increased, the cell concentration was diluted to 0.5×10⁶ cells/mL. Cell viability was assessed, and the cells were cultured continuously. NK cells were collected after 10-14 days of expansion culture.

### Example 2: Design of CAR sequence and preparation of its expression vector

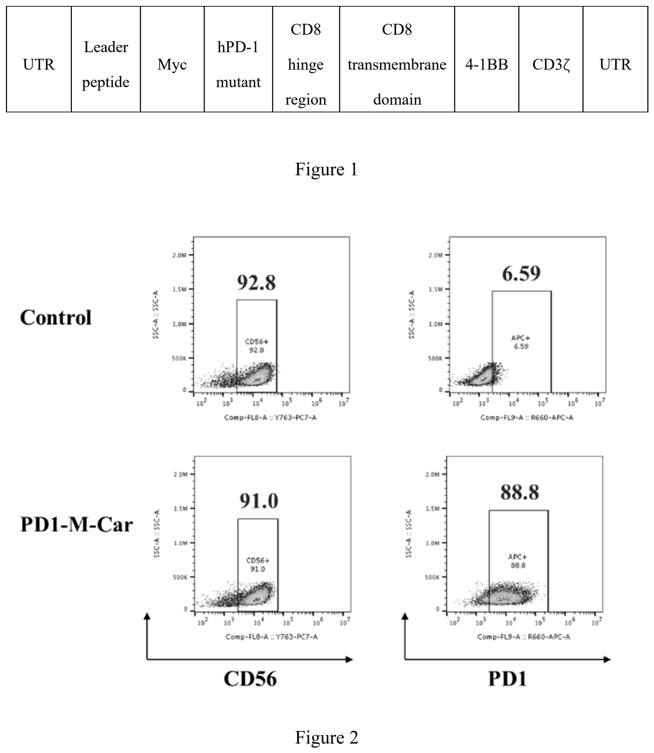

Encoding genes for UTR, CD8 signal peptide, human PD-1 mutant (PD1M), CD8α hinge region, CD8 transmembrane domain, 4-1BB signaling region, and CD3ζ signaling region were prepared, respectively. The above encoding genes for UTR, CD8 signal peptide, PD-1, CD8α hinge region, CD8 transmembrane domain, 4-1BB signaling region, and CD3ζ signaling region were sequentially ligated from the 5' terminus to the 3' terminus by PCR, obtaining the encoding gene of the chimeric antigen receptor PD1M-CAR. The schematic diagram of the PD1M-CAR is shown in Figure 1.

The amino acid sequence of the hPD-1 mutant is SEQ ID NO: 1:

FLDSPDRPWNPPTFSPALLVVTEGDNATFTCSFSNTSESFHVVWHRESPSG
QTDTLAAFPEDRSQPGQDCRFRVTQLPNGRDFHMSVVRARRNDSGTYVCGVISL
APKIQIKESLRAELRVTERRAEVPTAHPSPSPRPAGQFQTLV

The coding sequence of the chimeric antigen receptor PD1M-CAR is SEQ ID NO: 2:

gcattgcccgtgaccgccctgctgctgccactggccttgttgctccacgccgcgcggccaatgggatccTTC
CTGGACTCCCCTGACAGACCCTGGAACCCTCCTACCTTTTCCCCCGCTCTGCT
GGTGGTGACCGAGGGAGATAACGCCACCTTCACCTGCAGCTTTAGCAACACC
TCCGAGAGCTTCCACGTGGTGTGGCACAGAGAATCCCCCTCCGGACAGACCG
ACACCCTGGCTGCTTTCCCTGAAGACAGAAGCCAGCCCGGCCAGGATTGCAG
ATTTAGAGTGACCCAGCTGCCAAACGGCAGAGATTTTCACATGAGCGTGGTGC
GCGCTAGAAGAAACGACTCCGGCACCTATGTGTGCGGCGTGATCAGCCTGGC
TCCCAAAATCCAGATCAAGGAAAGCCTGAGAGCCGAGCTGAGAGTGACCGAG
AGAAGAGCCGAGGTGCCCACAGCTCACCCTAGTCCTAGCCCTAGGCCTGCTG
GACAGTTCCAGACCCTGGTGttcgtgccggtcttcctgccagcgaagcccaccacgacgccagcgcc
gcgaccaccaacaccggcgcccaccatcgcgtcgcagcccctgtccctgcgcccagaggcgtgccggccagcg
gcggggggcgcagtgcacacgagggggctggacttcgcctgtgatatctacatctgggcgcccttggccgggactt
gtggggtccttctcctgtcactggttatcaccaaacggggcagaaagaaactcctgtatatattcaaacaaccatttat
gagaccagtacaaactactcaagaggaagatggctgtagctgccgatttccagaagaagaagaaggaggatgtg
aactgagagtgaagttcagcaggagcgcagacgcccccgcgtaccagcagggccagaaccagctctataacga
gctcaatctaggacgaagagaggagtacgatgttttggacaagagacgtggccgggaccctgagatggggggaa
agccgagaaggaagaaccctcaggaaggcctgtacaatgaactgcagaaagataagatggcggaggcctaca
gtgagattgggatgaaaggcgagcgccggaggggcaaggggcacgatggcctttaccagggtctcagtacagcc
accaaggacacctacgacgcccttcacatgcaggccctgccccctcgctaa

The amino acid sequence of the chimeric antigen receptor PD1M-CAR is:

ALPVTALLLPLALLLHAARPMGSFLDSPDRPWNPPTFSPALLVVTEGDNATFT
CSFSNTSESFHVVWHRESPSGQTDTLAAFPEDRSQPGQDCRFRVTQLPNGRDF
HMSVVRARRNDSGTYVCGVISLAPKIQIKESLRAELRVTERRAEVPTAHPSPSPRP
AGQFQTLVFVPVFLPAKPTTTPAPRPPTPAPTIASQPLSLRPEACRPAAGGAVHTR
GLDFACDIYIWAPLAGTCGVLLLSLVITKRGRKKLLYIFKQPFMRPVQTTQEEDGCS
CRFPEEEEGGCELRVKFSRSADAPAYQQGQNQLYNELNLGRREEYDVLDKRRG
RDPEMGGKPRRKNPQEGLYNELQKDKMAEAYSEIGMKGERRRGKGHDGLYQGL
STATKDTYDALHMQALPPR* (SEQ ID NO: 3)

PD1WT-CAR expressing the wild-type PD-1 extracellular domain was obtained using the same method. For this PD1WT-CAR, all other parts are identical to those of PD1M-CAR except that its extracellular domain is the wild-type PD-1 extracellular domain. The amino acid sequence of this wild-type PD-1 extracellular domain is as follows (SEQ ID NO: 4):

FLDSPDRPWNPPTFSPALLVVTEGDNATFTCSFSNTSESFVLNWYRMSPSN
QTDKLAAFPEDRSQPGQDCRFRVTQLPNGRDFHMSVVRARRNDSGTYLCGAISL
APKAQIKESLRAELRVTERRAEVPTAHPSPSPRPAGQFQTLV

### (1) Construction of T7-PD1M-CAR Recombinant Plasmid

The PD1M-CAR gene was recombined into the T7 plasmid by PCR, and the recombinant plasmid was then transformed into E. coli competent cells DH5α. Positive clones were identified by PCR and sequencing. The PCR products were verified by gel electrophoresis and sequencing to confirm their size and sequence consistency with the target fragment. The plasmid was successfully constructed. Large quantities of the plasmid were obtained through *In vitro* extraction.

The same method was used to construct the T7-PD1WT-CAR recombinant plasmid.

### (2) mRNA synthesis

The sequences of PD1M-CAR and PD1WT-CAR were amplified by PCR from the T7 plasmids. The PCR products were then subjected to *In vitro* capping and polyadenylation to generate modified mRNA through transcription. The length and integrity of the RNA were verified by agarose gel electrophoresis. The purified RNA was dissolved in nuclease-free water/sodium citrate buffer at a concentration of 1-1.5 µg/µL for subsequent RNA electroporation experiments.

### Example 3: NK Cell Transfection

The mature NK cells obtained above were centrifuged to remove the medium, then counted using a hemocytometer. Each 20 µL of electroporation buffer contained 0.5-1 × 10⁶ NK cells. The cells were electroporated with the RNA prepared in Example 2 using a LONZA 4D electroporator, then cultured in complete NK medium (CST EXPAND MEDIUM #A5019001) to obtain NK cells expressing the chimeric antigen receptor PD1M-CAR (NK-PD1M-CAR cells). After 8 hours of culture, PD1M-CAR expression was detected by flow cytometry.

The results are shown in Figure 2. The maximum expression level of PD1M-CAR was 88.8%. Figure 2 indicates that the cell viability and CD56 expression of NK-PD1M-CAR cells were not affected.

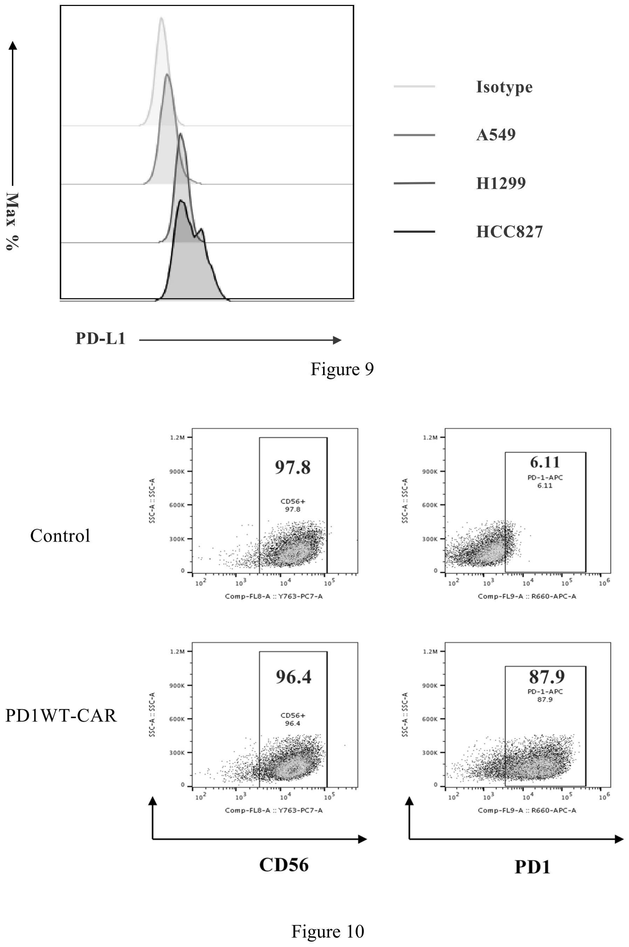

NK cells expressing PD1WT-CAR (NK-PD1WT-CAR cells) were obtained using the same method. The results are shown in Figure 10. The expression level of PD1WT-CAR was 87.9%. Figure 10 shows that there was no difference in cell viability or CD56 expression between NK-PD1WT-CAR cells and the cells before transfection.

### Example 4: In vitro killing effect of NK cells against PD-L1-overexpressing K562 tumor cell line

NK-PD1M-CAR cells prepared in Example 3, untreated NK cells (negative control group), and target cells (PD-L1-expressing K562 cells) were co-cultured *In vitro* at effector-to-target ratios of 5:1, 2.5:1, 1.25:1, and 0.625:1 in a 37°C, 5% CO₂ incubator. At 6 hours co-culture, the supernatants were collected by centrifugation at 250g, and the LDH content in each group was measured using an LDH assay kit to evaluate the killing ability of NK cells.

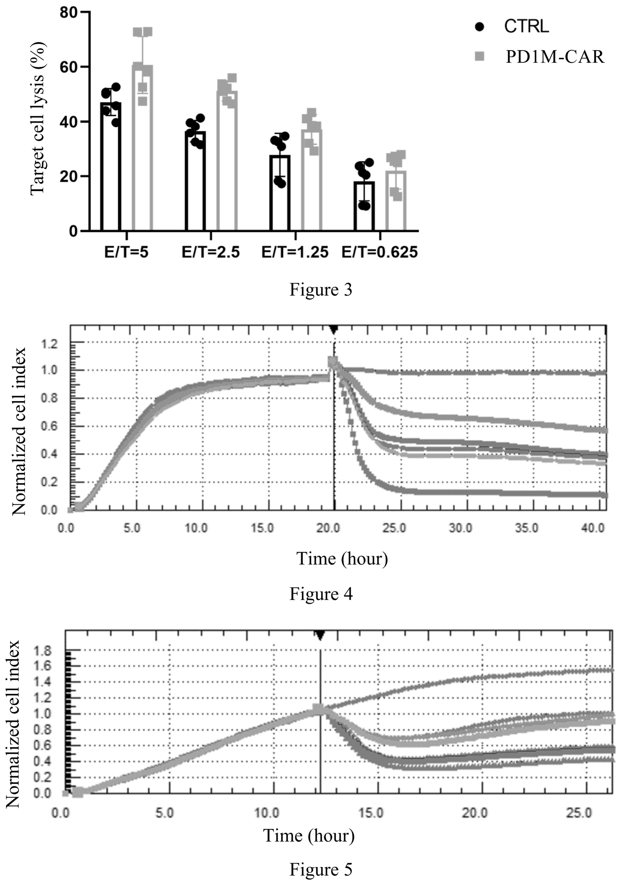

Each group had two replicates, and the NK cells were derived from three or more sample donors, with the results shown in Figure 3. As shown in Figure 3, the more NK-PD1M-CAR cells were added (i.e., the higher the effector-to-target ratio), the stronger their killing effect on tumor cells. The above results indicate that the NK-PD1M-CAR cells targeting PDL1 of the present invention have efficient and specific tumor killing ability, and can prevent tumor cells from escaping immune surveillance.

### Example 5: In vitro killing effect of NK cells on non-small cell lung cancer cell line A549

In this example, comparisons were made on the *In vitro* tumor-killing effects of NK-PD1M-CAR cells (prepared in Example 3), untreated NK cells (negative control group), NK lymphocytes expressing GFP (negative control group), NK lymphocytes overexpressing PD-1-WT (negative control group), and PD-1-M1 microbody used alone (negative control). Specific experimental methods include:

### 1. Day 1

The lung cancer cell line A549 was first digested with trypsin. After counting, the cells were resuspended in NK cell culture medium. Subsequently, following the operating manual of the RTCA instrument from Agilent Technologies, 10,000 tumor cells were evenly seeded into each well of a 96-well electronic microtiter plate. The target cells were cultured in the 96-well plate for 10 or 20 hours before proceeding to the next step.

### 2. Day 2

NK-PD1M-CAR cells prepared in Example 3, untreated NK cells (NK, negative control group), NK lymphocytes expressing GFP (NK-GFP, negative control group), and NK lymphocytes overexpressing PD-1-WT (NK-PD1WT-CAR, negative control group) were mixed with target cells at effector-to-target ratios of 1.25:1 and 0.625:1, respectively and cocultured at 37°C with 5% CO₂. PD-1-M1 microbody (HAC, SEQ ID NO: 5) was added at a final concentration of 0.25 µM. Real-time detection of the electrode plate reflected the death of target cells.

The experimental groups using NK-PD1M-CAR cells were as follows:
(1) A549;
(2) A549 + NK-GFP, E/T = 0.625;
(3) A549 + NK-GFP, E/T = 1.25;
(4) A549 + NK-GFP + HAC, E/T = 0.625;
(5) A549 + NK-GFP + HAC, E/T = 1.25;
(6) A549 + NK-PD1M-CAR, E/T = 0.625;
(7) A549 + NK-PD1M-CAR, E/T = 1.25.

Each group had two replicates.

The experimental groups using NK lymphocytes overexpressing PD-1-WT were as follows:
(1) A549;
(2) A549 + NK-GFP + HAC, E/T = 0.625;
(3) A549 + NK-GFP + HAC, E/T = 1.25;
(4) A549 + NK-PD1WT-CAR, E/T = 0.625;
(5) A549 + NK-PD1WT-CAR, E/T = 1.25;
(6) A549 + NK-PD1M-CAR, E/T = 0.625;
(7) A549 + NK-PD1M-CAR, E/T = 1.25.

Each group had two replicates.

The results are shown in Figures 4 and 5. As shown in Figure 4, compared with NK-GFP, NK-PD1M-CAR exhibits a stronger killing effect. HAC enhances the killing function of NK-GFP, but its effect is far lower than the killing ability of NK-PD1M-CAR.

It can be observed from Figure 5 that NK-PD1M-CAR exhibits a stronger killing effect compared to NK-GFP supplemented with HAC, and also exhibits a stronger killing effect compared to PD-1-WT-CAR.

### Example 6: In vitro killing effect of NK cells on non-small cell lung cancer cell line H1299

In this example, comparisons were made on the *In vitro* tumor-killing effects of NK-PD1M-CAR cells (prepared in Example 3), untreated NK cells (negative control group), NK lymphocytes expressing GFP (negative control group), and NK lymphocytes overexpressing PD-1-WT (negative control group). Specific experimental methods include:

### 1. Day 1

First, the lung cancer cell line H1299 was digested with trypsin. After counting, the cells were resuspended in NK cell culture medium. Subsequently, following the operating manual of the RTCA instrument from Agilent Technologies, 10,000 tumor cells were evenly seeded into each well of a 96-well electronic microtiter plate. The target cells were cultured in the 96-well plate for 20 hours before proceeding to the next step.

### 2. Day 2

NK-PD1M-CAR cells prepared in Example 3, NK lymphocytes expressing GFP (NK-GFP, negative control group), and NK lymphocytes overexpressing PD-1-WT (NK-PD1WT-CAR, negative control group) were mixed with target cells at an effector-to-target ratio of 1.25:1, and co-cultured at 37°C with 5% CO₂. PD-1-M1 microbody (HAC) was added at a final concentration of 0.25 µM. Real-time detection of the electrode plate reflected the death of target cells.

The experimental groups using NK-PD1M-CAR cells were as follows:
(1) H1299;
(2) H1299 + NK-GFP + HAC, E/T = 1.25;
(3) H1299 + NK-PD1WT-CAR, E/T = 1.25;
(4) H1299 + NK-PD1M-CAR, E/T = 1.25.

Each group had three replicates.

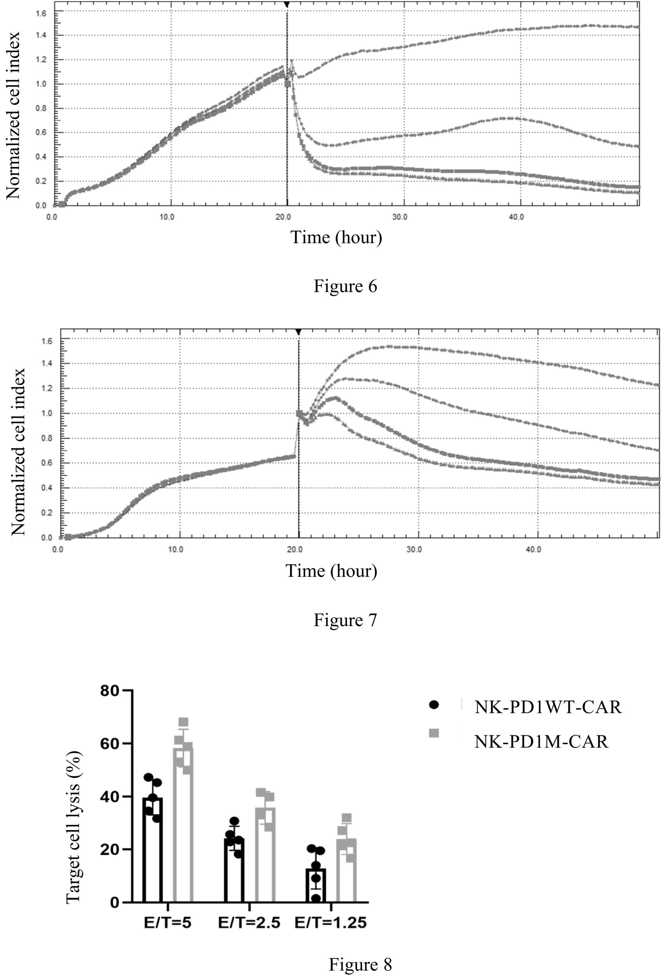

It can be observed from Figure 6 that NK-PD1M-CAR exhibits a stronger killing effect compared to NK-GFP supplemented with HAC, and also exhibits a stronger killing effect compared to PD-1-WT-CAR.

### Example 7: In vitro killing effect of NK cells on non-small cell lung cancer cell line HCC827

In this example, comparisons were made on the *In vitro* tumor-killing effects of NK-PD1M-CAR cells (prepared in Example 3), untreated NK cells (negative control group), NK lymphocytes expressing GFP (negative control group), and NK lymphocytes overexpressing PD-1-WT (negative control group). Specific experimental methods include:

### 1. Day 1

First, the lung cancer cell line HCC827 was digested with trypsin. After counting, the cells were resuspended in NK cell culture medium. Subsequently, following the operating manual of the RTCA instrument from Agilent Technologies, 10,000 tumor cells were evenly seeded into each well of a 96-well electronic microtiter plate. The target cells were cultured in the 96-well plate for 20 hours before proceeding to the next step.

### 2. Day 2

NK-PD1M-CAR cells prepared in Example 3, NK lymphocytes expressing GFP (NK-GFP, negative control group), and NK lymphocytes overexpressing PD-1-WT (NK-PD1WT-CAR, negative control group) were mixed with target cells at an effector-to-target ratio (E/T) of 1.25:1, and co-cultured at 37°C with 5% CO₂. PD-1-M1 microbody (HAC) was added at a final concentration of 0.25 µM. Real-time detection of the electrode plate reflected the death of target cells.

The experimental groups using NK-PD1M-CAR cells were as follows:
(1) HCC827;
(2) HCC827 + NK-GFP + HAC, E/T = 1.25;
(3) HCC827 + NK-PD1WT-CAR, E/T = 1.25;
(4) HCC827 + NK-PD1M-CAR, E/T = 1.25.

Each group had three replicates.

It can be observed from Figure 7 that NK-PD1M-CAR exhibits a stronger killing effect compared to NK-GFP supplemented with HAC, and also exhibits a stronger killing effect compared to PD-1-WT-CAR.

### Example 8: In vitro killing effect of NK cells on PDL1-overexpressing HCC827 tumor cell line

*In vitro,* NK-PD1M-CAR cells prepared in Example 3 and NK lymphocytes overexpressing PD-1-WT (NK-PD1WT-CAR, negative control group) were co-cultured with target cells (PDL1-expressing HCC827 cells) at effector-to-target ratios of 5:1, 2.5:1, and 1.25:1, at 37°C with 5% CO₂. At 4 hours after culture, supernatants were collected after centrifugation at 250g, and LDH content in the supernatants was measured using an LDH kit to evaluate the killing ability of NK cells.

Each group had two replicates, and NK cells were derived from three or more sample donors. Results are shown in Figure 8. As shown in Figure 8, the more NK-PD1M-CAR cells added (i.e., the higher the effector-to-target ratio), the stronger the killing effect on tumor cells. NK-PD1M-CAR cells exhibited significantly stronger killing activity compared to NK-PD1WT-CAR cells. The above results indicate that the NK-PD1M-CAR cells targeting PDL1 of the present invention have efficient and specific tumor killing ability, and can prevent tumor cells from escaping immune surveillance.

### Example 9: PDL1 expression in target cells

According to the conventional cell antibody staining method, A549, H1299, and HCC827 cells were digested with trypsin respectively, centrifuged at 300g for 5 minutes, then resuspended in PBS to obtain single-cell suspensions. Each type of cell was counted, and 1 million cells were taken into 5ml round bottom glass tubes. 2-3ml of PBS was added to wash the cells, followed by centrifugation at 300g for 5 minutes; the supernatant was then discarded. 5µl of isotype-APC (Biolegend #402206) and PDL1-APC (Biolegend #329708) were added respectively, and the cells were stained at 4°C for 30 minutes before detection using a flow cytometer (CytoFLEX5).

The detection results are shown in Figure 9. The results show that A549, H1299, and HCC827 all express PDL1, and the expression levels of PDL1 are: HCC827>H 1299>A549.

### Example 10: in vivo killing effect of PD-1-M1 CAR NK cells

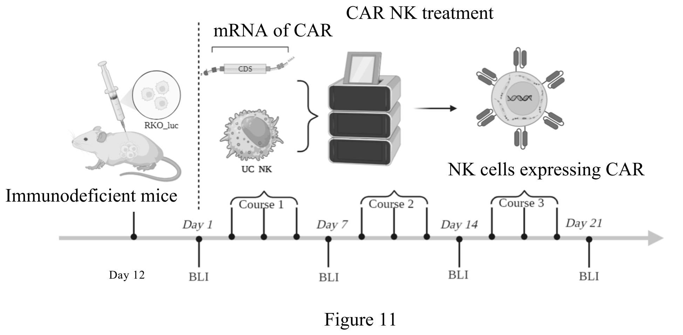

In this example, humanized B-NDG mice intraperitoneally inoculated with RKO tumor cells were used to verify the *in vivo* killing effect of PD-1-M1 CAR NK cells. Tumor cells grow slowly in the abdominal cavity, 12 days after RKO cell inoculation, obvious tumor formation was observed, which was used to simulate the real tumor formation process in humans. The electroporation method described in Example 3 was used to obtain NK-PD1M-CAR cells and NK-PD1WT-CAR cells. These cells were reinfused 2-3 times per week for three treatment cycles, with non-electroporated NK cells used as control. At the same time, cytokines IL2 and IL15 were injected before NK cell reinfusion to create a more suitable growth environment for NK cells. Live imaging of small animals was performed on the 7th, 14th and 21st days after reinfusion. The experimental method is shown in Figure 11.

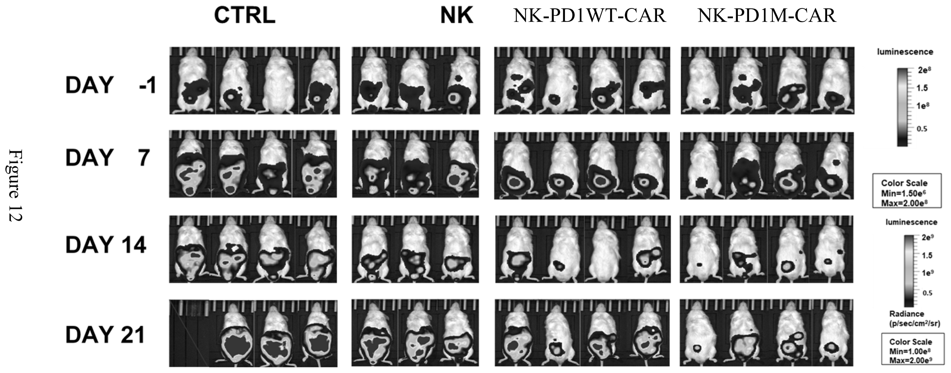

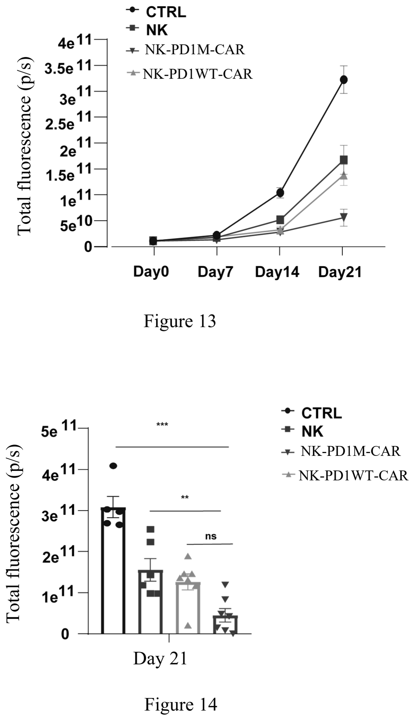

After the first week of treatment, obvious metastatic lesions appeared in the control group inoculated with tumor only. Compared with the NK cell and NK-PD1WT-CAR cell groups, the tumor growth in the NK-PD1M-CAR cell group was significantly inhibited. After the second course of treatment, compared with the control group inoculated with tumor only, the reinfusion of NK cells alone could limit the growth of RKO colon cancer to a certain extent, while the NK-PD1WT-CAR cell group and NK-PD1M-CAR cell group showed a more obvious inhibitory effect on tumor growth (Figures 12 and 13). According to the statistics of the fluorescent signal of mice, at the end of the third course of treatment, in the late stage of tumor growth, the tumor growth of mice in the control group was very rapid, and compared with the NK-reinfusion, the NK-PD1WT-CAR cell group and the NK-PD1M-CAR cell group significantly slowed down the tumor growth rate (Figure 14).

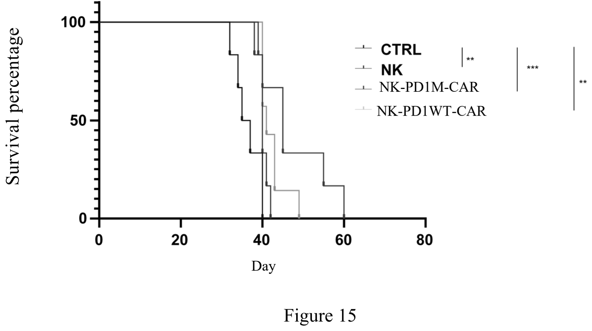

After the above three courses of treatment, the death of tumor-bearing mice under various treatment conditions was observed and counted. In the late stage of tumor inoculation, some treated mice developed severe ascites. According to the ethical regulations for mice, mice that gained 10% of their weight after ascites must be euthanized. The survival curve of the mice is as follows. Compared with the control group inoculated with tumors only, the life span of tumor-bearing mice in the NK-PD1WT-CAR cell group and the NK-PD1M-CAR cell group was significantly prolonged (Figure 15).

## Claims

1. A polypeptide comprising the amino acid sequence shown by SEQ ID NO: 1, or consisting of the amino acid sequence shown by SEQ ID NO: 1.

2. A chimeric antigen receptor comprising, from the N-terminus to the C-terminus, an optional signal peptide, the polypeptide according to claim 1, a hinge region, a transmembrane domain, an intracellular co-stimulatory signaling domain, and an intracellular signaling domain, which are sequentially connected.

3. The chimeric antigen receptor according to claim 2, wherein:
the signal peptide is selected from the group consisting of: a CD8 signal peptide, a CD28 signal peptide, or a CD4 signal peptide; preferably, the signal peptide is a CD8 signal peptide; preferably, the amino acid sequence of the CD8 signal peptide is shown by amino acid residues 1-20 of SEQ ID NO: 3; and/or
the hinge region is selected from the group consisting of: a CD8α hinge region, an IgD hinge region, an IgG1 Fc CH2CH3 hinge region, or an IgG4 Fc CH2CH3 hinge region; preferably, the hinge region is a CD8α hinge region; preferably, the amino acid sequence of the CD8α hinge region is shown by amino acid residues 171-225 of SEQ ID NO: 3; and/or
the transmembrane domain is selected from the group consisting of: a CD28 transmembrane domain, a CD8 transmembrane domain, a CD3ζ transmembrane domain, a CD134 transmembrane domain, a CD137 transmembrane domain, an ICOS transmembrane domain, or a DAP10 transmembrane domain; preferably, the transmembrane domain is a CD8 transmembrane domain; preferably, the amino acid sequence of the CD8 transmembrane domain is shown by amino acid residues 226-246 of SEQ ID NO: 3; and/or
the intracellular co-stimulatory signaling domain is an intracellular domain of a co-stimulatory signaling molecule, preferably selected from the group consisting of: the intracellular domains of CD28, CD134/OX40, CD137/4-1BB, lymphocyte-specific protein tyrosine kinase, inducible T-cell co-stimulator, or DNAX-activating protein 10; preferably, the intracellular co-stimulatory signaling domain is the intracellular domain of 4-1BB; preferably, the amino acid sequence of the intracellular domain of 4-1BB is shown by amino acid residues 247-288 of SEQ ID NO: 3; and/or
the intracellular signaling domain is a CD3ζ intracellular signaling domain or an FcεRIγ intracellular signaling domain; preferably, the intracellular signaling domain is a CD3ζ intracellular signaling domain; preferably, the amino acid sequence of the CD3ζ intracellular signaling domain is shown by amino acid residues 289-400 of SEQ ID NO: 3.

4. The chimeric antigen receptor according to claim 3, wherein the chimeric antigen receptor comprises, in sequence from the N-terminus to the C-terminus, a CD8 signal peptide, the polypeptide according to claim 1, a CD8α hinge region, a CD8 transmembrane domain, an intracellular domain of 4-1BB, and a tyrosine activation motif of CD3ζ;
preferably, the amino acid sequence of the chimeric antigen receptor consists of amino acid residues 1-20 of SEQ ID NO: 3, SEQ ID NO: 1, and amino acid residues 171-400 of SEQ ID NO: 3, sequentially linked from the N-terminus to the C-terminus; or the amino acid sequence of the chimeric antigen receptor is as shown by SEQ ID NO: 3.

5. A nucleic acid molecule encoding the polypeptide according to claim 1 or the chimeric antigen receptor according to any one of claims 2 to 4; preferably, the nucleic acid molecule is a DNA molecule or an RNA molecule;
preferably, the nucleic acid molecule is the coding sequence for the polypeptide according to claim 1, and its nucleotide sequence is shown by nucleotides 70-510 of SEQ ID NO: 2;
preferably, the nucleic acid molecule sequentially comprises, from the 5' terminus to the 3' terminus: the nucleotide sequence shown by nucleotides 1-60 of SEQ ID NO: 2 and the nucleotide sequence as shown by nucleotides 70-1200 of SEQ ID NO: 2, or its nucleotide sequence is shown by SEQ ID NO: 2.

6. A nucleic acid construct comprising the nucleic acid molecule according to claim 5;
preferably, the nucleic acid construct is an expression cassette, which further comprises regulatory sequences in addition to the nucleic acid molecule; or the nucleic acid construct is a vector, including an expression vector and an integration vector for integrating the nucleic acid molecule into the genome of a host cell; preferably, the vector is a transposon vector, more preferably, the transposon vector is a eukaryotic expression vector containing a transposable element selected from the group consisting of: piggybac, sleeping beauty, Frog Prince, Tn5, or Ty.

7. A host cell comprising the nucleic acid construct according to claim 6, and/or expressing the polypeptide according to claim 1 or the CAR according to any one of claims 2 to 4; preferably, the host cell is an NK cell.

8. A composition or kit comprising the vector according to claim 6 and an optional reagent for transfection.

9. A pharmaceutical composition comprising the NK cells according to claim 7 and a pharmaceutically acceptable carrier or excipient.

10. Use of the polypeptide according to claim 1, the chimeric antigen receptor according to any one of claims 2 to 4, the nucleic acid molecule according to claim 5, the nucleic acid construct according to claim 6, the host cell according to claim 7, or the pharmaceutical composition according to claim 9 in the manufacture of a medicament for treating or preventing PD1- or PDL1-mediated cancer; preferably, the cancer is selected from the group consisting of: gastric cancer, lung cancer (e.g., non-small cell lung cancer), liver cancer, intrahepatic cholangiocarcinoma, colon cancer, pancreatic cancer, ovarian cancer, breast cancer, cervical cancer, head and neck squamous cell carcinoma, nasopharyngeal carcinoma, esophageal cancer, bladder cancer, renal cell carcinoma, skin cancer, and oral squamous cell carcinoma.
